Europäisches Patentamt
European Patent Office
Office européen des brevets

(19)

(11) Publication number: **0 394 950 A1**

(12) **EUROPEAN PATENT APPLICATION**

(21) Application number: 90107721.4

(22) Date of filing: 24.04.90

(51) Int. Cl.[5]: C07D 219/10, C07D 221/16, A61K 31/44, A61K 31/47

(30) Priority: 25.04.89 JP 106387/89
28.04.89 JP 110846/89

(43) Date of publication of application:
31.10.90 Bulletin 90/44

(84) Designated Contracting States:
AT BE CH DE DK ES FR GB GR IT LI LU NL SE

(71) Applicant: SUMITOMO PHARMACEUTICALS COMPANY, LIMITED
2-8, Dosho-Machi 2-Chome, Chuo-Ku
Osaka-Shi Osaka-Fu(JP)

(72) Inventor: Kitano, Masahumi
4-2-304, Ryodocho
Nishinomiya-shi(JP)
Inventor: Ohuchi, Renzo
10-3-326, Sonehigashinocho-2-chome
Toyonaka-shi(JP)
Inventor: Ono, Keiichi
10-4, Momoyamadai-3-cho
Sakai-shi(JP)

(74) Representative: Vossius & Partner
Siebertstrasse 4 P.O. Box 86 07 67
D-8000 München 86(DE)

(54) **Pyridine derivatives.**

(57) A pyridine derivative of the formula

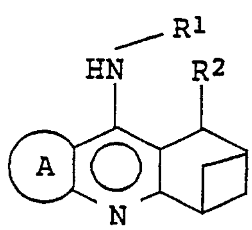

(I)

wherein

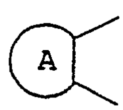

,

$R^1$ and $R^2$ are each specific groups, or pharmaceutically acceptable acid addition salt thereof is a novel compound which has an excellent pharmacological activity for increasing acetylcholine level in the brain without adverse side effects. Accordingly, those compounds are useful for the treatment of dementia of Alzheimer's type.

EP 0 394 950 A1

## PYRIDINE DERIVATIVES

This invention relates to pyridine derivatives and pharmaceutically acceptable acid addition salts thereof, and to medical use thereof. More particularly, it relates to pyridine derivatives and pharmaceutically acceptable acid addition salts thereof which have a pharmacological activity for increasing acetylcholine level in the brain due to their inhibitory action on acetylcholinesterase and which are useful for the treatment of various memory dysfunctions characterized by decreased function, such as Alzheimer's disease and senile dementia of Alzheimer's type, and to medical use thereof.

It has been known that acetylcholine level is decreased in the brain of patients suffering from Alzheimer's disease (In the Aging Brain, Berlin, 1982, p. 140). It has been examined whether or not physostigmine, which is an acetylcholinesterase inhibitor, is efficacious for the treatment of senile dementia [Neurology, 8, p. 397 (1978)]. Additionally, it has been reported that 9-amino-1,3,3,4-tetrahydroacridinol derivatives (US-A- 4,631,286, 4,695,573, and 4,839,364), 9-amino-2,3,5,6,7,8-hexahydro-1H-cyclopenta (b) quinoline monohydrate hydrochloride (US-A-4,550,113 and 4,735,953), 4-aminopyridine compounds (WO-A-89/02,739 and WO-A-89/02,740), 4-amino-dihydropyridine derivatives (EP-A-326,134) and quinoline derivatives (EP-A-268,871) each have an inhibitory activity against acetylcholinesterase.

It has been reported that 9-acylaminotetrahydroacridine derivatives (EP-A-319,429) have an activity for activating decreased cholinergic nervous systems and can be therefore used as a memory enhancer in the treatment of memory disorders, e.g. Alzheimer's disease and senile dementia.

It has been reported that 9-amino-1,4-ethano-1,2,3,4-tetrahydroacridine and its related compounds (EP-A-278,499) can be used for the treatment of various memory dysfunctions characterized by decreased cholinergic function, such as Alzheimer's disease.

Further, it has been reported that aminoacridine derivatives exhibit analgesic, sedative, and antidepressant activities (US-A-3,232,945).

It has been known that a combination of tacrine (9-amino-1,2,3,4-tetrahydroacridine) having an inhibitory activity on acetylcholinesterase with lecithin are useful for the treatment of Alzheimer's disease by intravenous injection. [Neurobiology Aging, 4, p. 139 (1983)].

Tacrine has been also reported to induce improved performance in patients afflicted with Alzheimer's disease at psychological tests [The New England Journal of Medicine, 315, 1241-1245 (1986) and US-A-4,816,456]. On the other hand, tacrine has been also reported to have hepatoxicity as side effect [The Lancet, 729, (1989)].

It has been reported that an acetylcholinesterase inhibitor is useful for the treatment of various memory dysfunctions characterized by decreased cholinergic function, because it has an activity for increasing acetylcholine level in the brain. However, it has been also known that acetylcholinesterase inhibitors have cholinergic peripheral side effects (e.g. salivation, diarrhea, lacrimation etc.) (H. Itoh and Y. Misu, Pharmacology, p. 184, Eikodo). Heretofore, an acetylcholinesterase inhibitor without adverse side effects has not been found yet.

Accordingly, a primary object of the present invention is to prove novel pyridine derivatives having an inhibitory activity against acetylcholinesterase and also exhibiting effects on amnestic models in several animals without adverse side effects and therefore useful for the treatment of senile dementia of Alzheimer's type.

The present invention is directed to novel pyridine derivatives represented by formula (I),

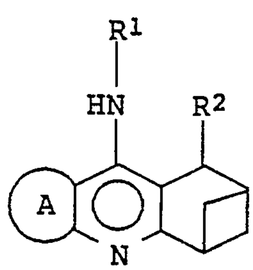

(I)

wherein $R^1$ is hydrogen or aralkyl, and $R^2$ and

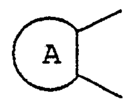

are defined as in the following (1) or (2):

(1) 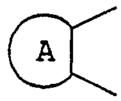 is 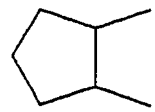 and $R^2$ is hydrogen; or

(2) 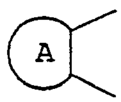 is 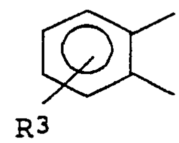

wherein $R^3$ is hydrogen, halogen or lower alkoxy, and $R^2$ is hydroxy; and pharmaceutically acceptable acid addition salts thereof.

The present invention is further directed to a pharmaceutical composition which comprises at least one of the pyridine derivatives of formula (I) and pharmaceutically acceptable acid addition salts thereof as an active ingredient together with a pharmaceutically acceptable carrier or diluent.

. Thirdly, the present invention is directed to the use of at least one of the pyridine derivatives of formula (I) and pharmaceutically acceptable acid addition salts thereof as a pharmaceutically active substance for the treatment of senile dementia of Alzheimer's type.

In the definitions given above, the term "halogen" includes fluorine, chlorine, bromine, and iodine. The term "lower alkoxy" means a straight or branched chain alkoxy group having 1 to 4 carbon atoms (e.g. methoxy, ethoxy, n-propoxy, isopropoxy, n-butoxy and sec-butoxy ). The term "aralkyl" means phenylalkyl or (substituted phenyl)alkyl having 7 to 13 carbon atoms. The term "alkyl" in "phenylalkyl" and "-(substituted phenyl)alkyl" means lower straight alkylene group having 1 to 4 carbon atoms (e.g. methylene, ethylene, propylene and butylene). The substituted phenyl in "(substituted phenyl)alkyl" is a phenyl group having one substituent selected from the group consisting of halogen (e.g. fluorine, chlorine, bromine and iodine), lower alkyl which includes a straight or branched chain alkyl group having 1 to 4 carbon atoms (e.g. methyl, ethyl, n-propyl, isopropyl, n-butyl, and sec-butyl ), and lower alkoxy which includes a straight or branched chain alkoxy group having 1 to 4 carbon atoms (e.g. methoxy, ethoxy, n-propoxy, isopropoxy, n-butoxy, and sec-butoxy ). Examples of such aralkyls include benzyl, phenethyl, 2-(4-methoxyphenyl)ethyl, 2-(4-methylphenyl)ethyl, 2-(4-fluorophenyl)ethyl, 3-phenylpropyl, 4-phenylbutyl, and 4-(4-fluorophenyl)butyl.

The term "pharmaceutically acceptable acid addition salts" includes the salts formed from inorganic acids such as hydrochloric acid, hydrobromic acid, sulfuric acid, nitric acid, phosphoric acid and perchloric acid; or organic acids such as tartaric acid, succinic acid, maleic acid, fumaric acid and citric acid.

The pyridine derivatives (I) have an inhibitory action on acetylcholinesterase and show an effect on the amnestic model in several animals without adverse side effects.

According to the present invention, the pyridine derivatives (I) can be prepared, for instance, by the following synthetic routes.

(1) The first route (A):

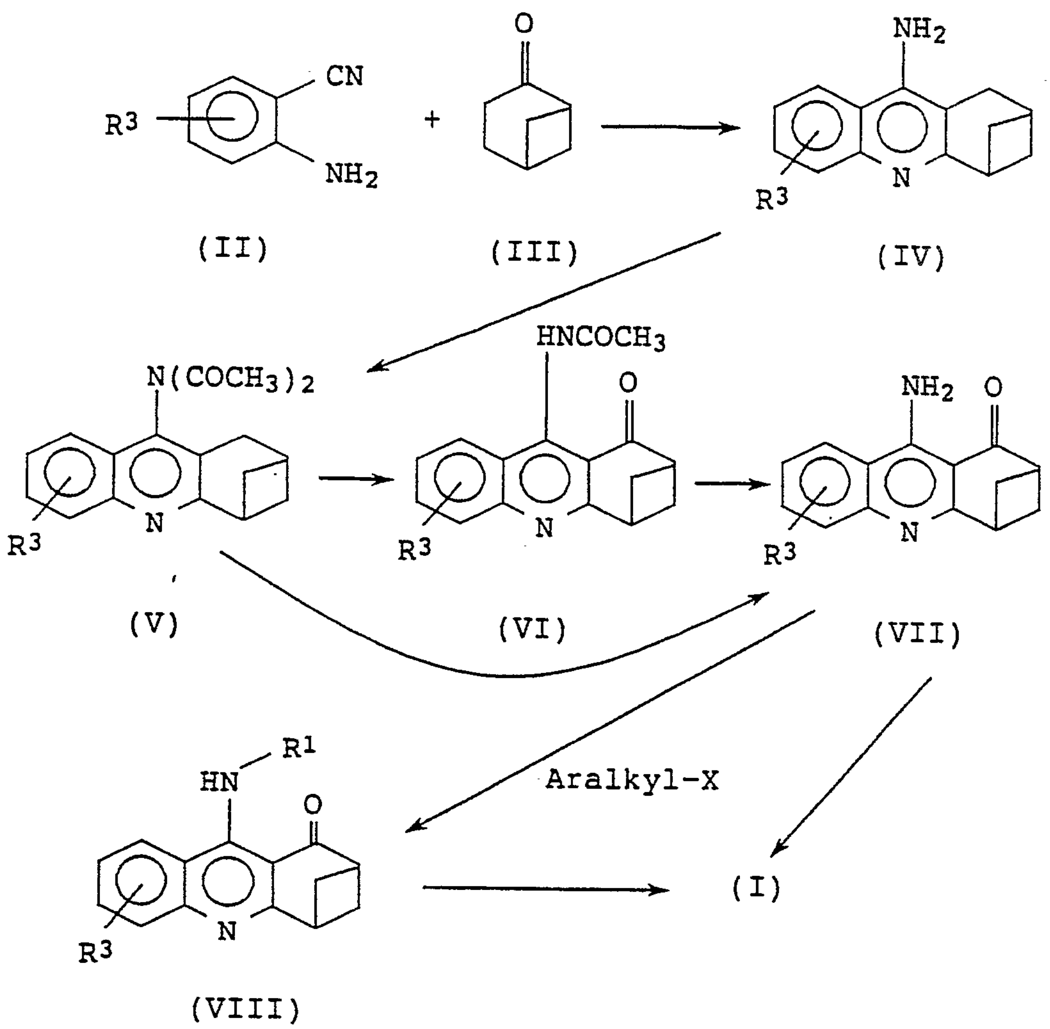


In the above route, $R^3$ is hydrogen, halogen, or lower alkoxy, $R^1$ is hydrogen or aralkyl, and X is bromine or chlorine.

A synthetic method for the preparation of the compound (IV) is known (EP-A-268,871). The compound (V) can be obtained by the reaction of the compound (IV) with acetic anhydride in pyridine at a temperature in the range of 90 °C to under reflux. The oxidation of the compound (V) to the compound (VI) and/or to the compound (VII) is carried out according to the method described in Shin Jikken Kagaku Koza (New Experimental Chemistry), Vol. 15, "Oxidation and Reduction [I-1]", Japanese Chemical Association. For example, the oxidation is carried out by the use of chromic anhydride of chromate (VI) compounds (e.g. sodium dichromate dihydrate ), in an inert solvent at the temperature in the range of ice-cooling to under reflux. The inert solvent is, for instance, acetic acid, acetic acid-water or acetic acid-acetic anhydride.

The reaction solvent used and the range of reaction temperature may be varied depending upon the oxidizers.

The compound (VI) is subjected to hydrolysis by 50% sulfuric acid to yield the compound (VII). The aralkylation of the compound (VII) can be carried out according to the conventional method as described in, for example, Shin Jikken Kagaku Kouza (New Experimental Chemistry), Vol. 14, p. 1332, "A reaction and synthesis of organic compound", Japanese Chemical Association. Those reactions can be also carried out according to the same method as described in US-A-4,631,286 wherein there is used a strong aqueous alkaline solution such as 50% aqueous sodium hydroxide solution, in organic solvent such as dichloromethane and toluene, as a two phase-reaction system containing phase-transferring catalyst such as tetrabutylammonium hydrogen sulfate.

The reduction of the compound (VII) or (VIII) can be carried out in the same method as described in US-A- 4, 631,286. For example, the reduction is performed by the use of a suitable metal hydride in an organic solvent such as an ethereal solvent including tetrahydrofuran or ether, at the temperature in the range of -20 to 20 °C.

(2) The second route (B):

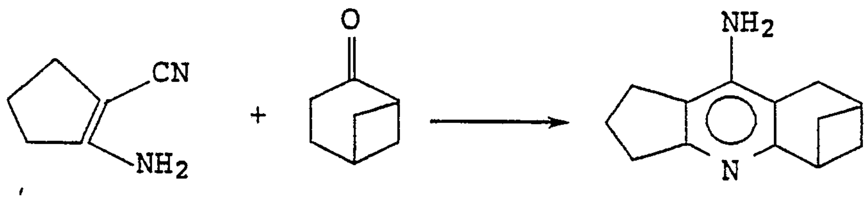

(IX) (III) (I')

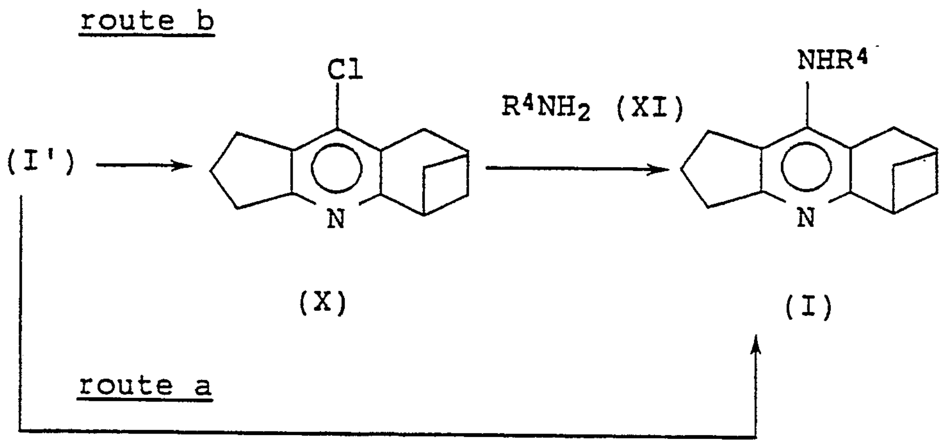


In the above route, $R^4$ is aralkyl.

The preparation of the compound (I′) can be conducted by reacting the compound (IX) with the compound (III) in the presence of Lewis acid. As the Lewis acid, there may be exemplified anhydrous aluminum chloride or anhydrous aluminum bromide. Suitable solvents for the reaction of the compound (IX) with the compound (III) include 1,2-dichloroethane and nitrobenzene. The reaction is conducted at a temperature in the range of 50 to 150 °C, preferably 70 to 130 °C.

The aralkylation of the compound (I′) can be conducted according to the same method as mentioned above in the alkylation of the compound (VII) at the first route (route a). The aralkylation can be also effected according to the conventional method (route b) as follows. The synthesis of the compound (X) from the compound (I′) can be accomplished by reacting the compound (I′) with sodium nitrite in the presence of hydrochloric acid at a temperature in the range of 0 to 5 °C [Organic Synthesis Collective Vol. III, p. 295], followed by reacting copper (I) chloride at a temperature in the range of 70 to 90 °C [Organic Synthesis Collective Vol. II. p. 130]

The compound (I) can be obtained by reacting the compound (X) with amines (XI) according to the same method as described in Indian Journal of Chemistry, Section B, 16B (2), p. 156 (1978). The reaction is conducted at the temperature in the range of 100 to 150 °C in phenol.

A salt-forming reaction to prepare the pharmaceutically acceptable acid addition salts of the compound (I) can be conducted according to the conventional method wherein the obtained compound (I) is allowed to contact with the organic acid or inorganic acid in an appropriate solvent such as alcohols (e.g. methanol, ethanol or isopropanol ) or ether.

Phyridine derivatives (I) and their acid addition salts can be administrated orally or parenterally in the form of conventional pharmaceutical preparations such as tablets, capsules, syrups, and suspensions. Alternatively, they can be administered parenterally by injection in the form of solutions, emulsions, etc. They may be directly applied to rectum in the form of suppository. The preparations may contain physiologically acceptable carriers, excipients, activators, binding agents, stabilizers, etc. In the case of injections, physiologically acceptable buffers, solubilizing agents or isotonic agents may be incorporated therein. The daily dose may vary depending upon the symptom of disease, the age and body weight of

patients, the administration route, etc., and the normal dose to a human adult is between 1 mg and 500 mg, preferably between 5mg and 300 mg divided into several portions per day.

The following compounds are preferably provided with the present invention :

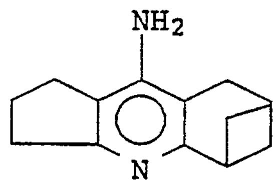
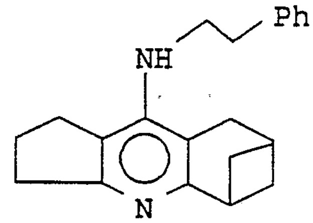
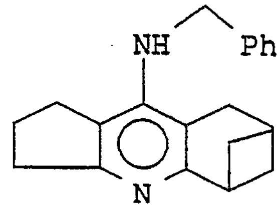

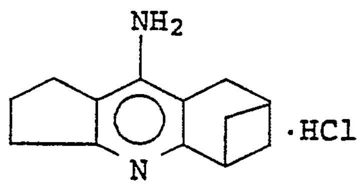
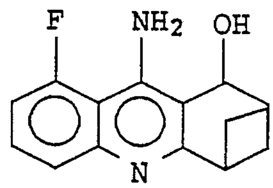
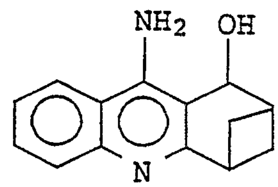

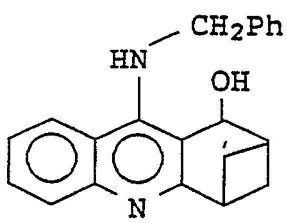
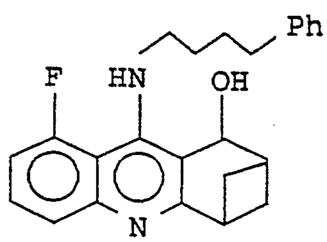
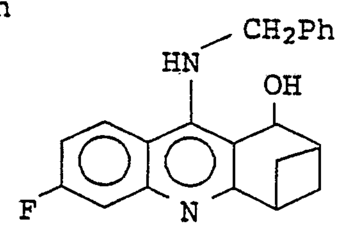

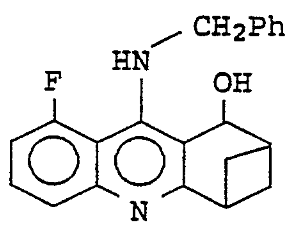
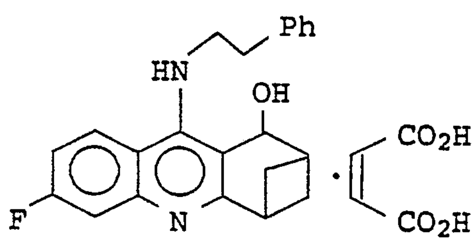

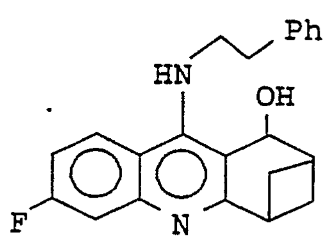
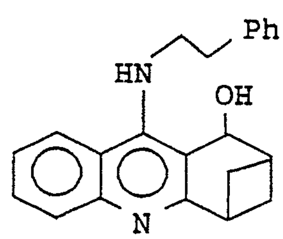
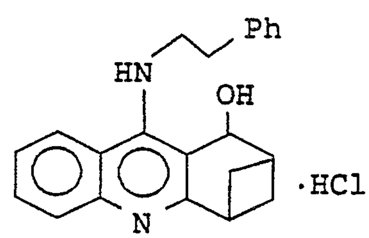

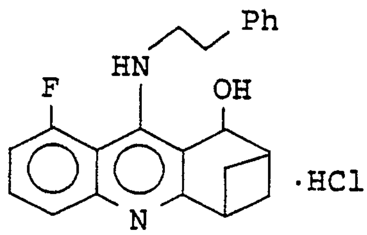
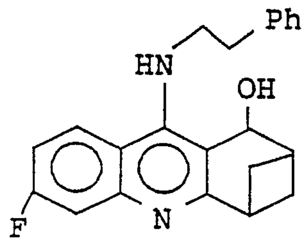
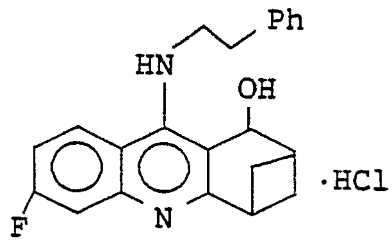

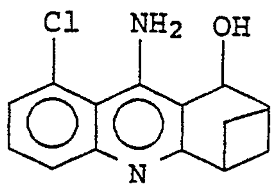
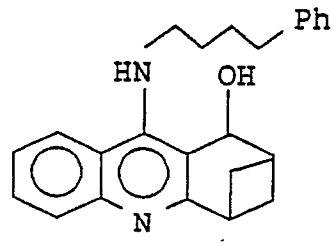
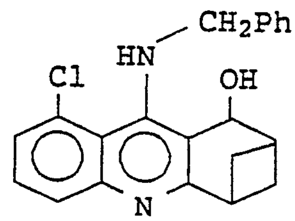

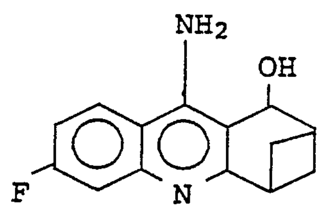
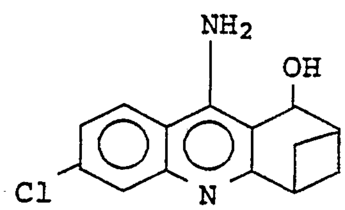

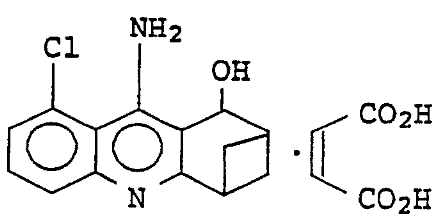
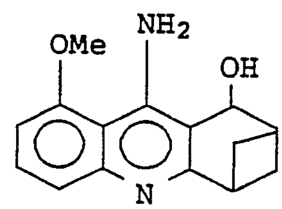

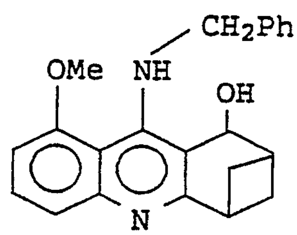
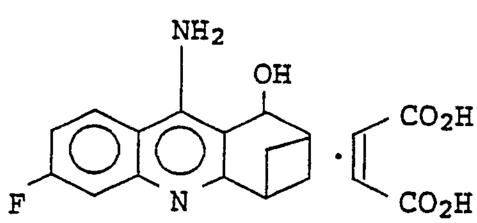

Practically and presently preferred embodiments of the invention are illustratively shown in the following Reference Examples and Examples, which are not intended to limit the scope of the invention thereto.

Reference Example 1

Preparation of 9-diacetylamino-8-fluoro-2,4-methano-1,2,3,4-tetrahydroacridine:

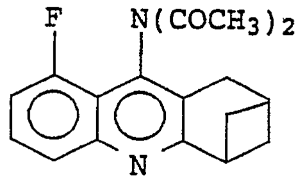

A mixture of 9-amino-8-fluoro-2,4-methano-1,2,3,4-tetrahydroacridine (100 g), acetic anhydride (300 ml) and pyridine (500 ml) was heated for 15 hours at 100 - 110 °C. Thereafter, reaction solvent was evaporated, and then xylene (500 ml) was added to the residue. Evaporation of xylene gave an oil which was subjected to column chromatography on silica gel. Elution with n-hexane-ethyl acetate (1:1) gave 9-diacetylamino-8-fluoro-2,4-methano-1,2,3,4-tetrahydroacridine.

NMR ($CDCl_3$, TMS): 1.58-1.66 (2H, m), 2.32 (6H, s), 2.61-2.69 (2H, m), 2.85-2.92 (3H, m), 3.51-3.57 (1H, m), 7.13-7.21 (1H, m), 7.55-7.63 (1H, m), 7.86-7.89 (1H, m).

Reference Example 2

Preparation of 9-diacetylamino-3,4-dihydro-8-fluoro-2,4-methanoacridin-1(2H)-one:

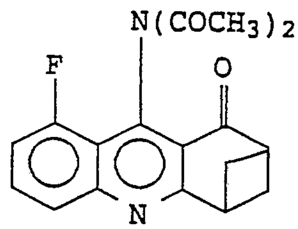

To a mixture of 9-diacetylamino-8-fluoro-2,4-methano-1,2,3,4-tetrahydroacridine (31 g) obtained in Reference Example 1 and acetic acid (250 ml), a solution of chromium (VI) oxide (40 g) in water (20 ml) and acetic acid (110 ml) was added dropwise at a temperature in the range of 10 to 17 °C, followed by stirring for 2 hours at the same temperature. Thereafter, water was added to the reaction mixture, and then the mixture was extracted with ether. The extract was washed successively with water, an aqueous sodium hydrogen carbonate solution and dried over anhydrous magnesium sulfate. Evaporation of the solvent gave a residue, which was purified by column chromatography on silica gel. There was obtained 9-diacetylamino-3,4-dihydro-8-fluoro-2,4-methanoacridin-1(2H)-one.

Reference Example 3

Preparation of 9-amino-3,4-dihydro-8-fluoro-2,4-methanoacridin-1(2H)-one:

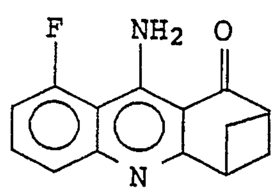

The acridine derivative obtained in Reference Example 2 was added to 50% sulfuric acid, followed by stirring at room temperature. An aqueous sodium hydroxide solution was added to the reaction mixture. The resulting mixture was alkalified. The resulting crystalline product was filtered with suction, followed by purifying by column chromatography on silica gel to give 9-amino-3,4-dihydro-8-fluoro-2,4-methanoacridin-1(2H)-one.
NMR ($CDCl_3$, TMS): 2.40-2.47 (2H, m), 2.85-2.97 (2H, m), 3.11-3.18 (1H, m), 3.45-3.52 (1H, m), 6.85-7.08 (2H, m), 7.54-7.68 (2H, m), 9.57 (1H, br).

Reference Example 4

Preparation of 9-amino-3,4-dihydro-8-fluoro-2,4-methanoacridin-1(2H)-one:

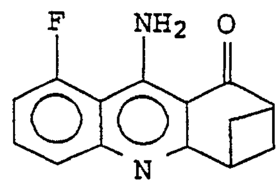

The acridine derivative (31.2 g) obtained in Reference Example 1 was added to acetic acid (300 ml) and heated at 130-140 °C. Sodium dichromate dihydrate (185.72 g) was added to the mixture by small portions. After the reaction mixture was heated under reflux for three hours, acetic anhydride (50 ml) was added dropwise to the reaction mixture, followed by refluxing. After cooling, an aqueous sodium hydroxide solution was added to the reaction mixture. The resulting mixture was alkalified and ethyl acetate (500 ml) was added, followed by stirring for 10 minutes. After filtration, the mixture was extracted three times with ethyl acetate. The extracts were combined and washed with a saturated aqueous sodium chloride solution and dried over anhydrous magnesium sulfate. Evaporation of ethyl acetate gave a crude crystalline product, which was recrystallized from ethyl acetate to give 9-amino-3,4-dihydro-8-fluoro-2,4-methanoacridin-1(2H)-one, of which NMR spectra data was same as the compound which was obtained in Reference Example 3.

Reference Example 5

Preparation of 9-chloro-2,3,5,6,7,8-hexahydro-1H-5,7-methanocyclopenta (b) quinoline:

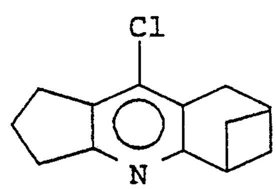

The pyridine derivative (9.5 g) obtained in Example 3 mentioned hereinafter was added to 28% hydrochloric acid (48 ml) and cooled to 0-5 °C with stirring. To the suspension, an aqueous solution (20 ml) of sodium nitrite (3.9 g) was added dropwise at the temperature in the range of 0 to 5 °C (hereinafter this mixture is abbreviated as to DIAZO). The mixture of copper (II) sulfate pentahydrate (21 g), sodium chloride (8.1 g) and water (43 ml) was heated at 70-80 °C with stirring. To the mixture, an aqueous solution (24 ml) of sodium metabisulfite (3 g) and sodium hydroxide (2 g) was added, followed by stirring for 30 minutes at 70-80 °C. After cooling to 0-5 °C, DIAZO was added to this mixture. 28% Hydrochloric acid (60 ml) was added to the reaction mixture, followed by stirring for 4 hours at room temperature. An aqueous sodium hydroxide solution (20%) was added to the reaction mixture. The resulting mixture was alkalified and extracted three times with ethyl acetate. The extract was washed with water and dried over anhydrous magnesium sulfate. Evaporation of the solvent gave a residue, which was subjected to column chromatography on silica gel. Elution with n-hexane : ethyl acetate = 7 : 3 gave 9-chloro-2,3,5,6,7,8-hexahydro-1H-5,7-methanocyclopenta (b) quinoline.

NMR ($CDCl_3$, TMS): 1.45-1.52 (2H, m), 2.08-2.19 (2H, m), 2.45-2.55 (2H, m), 2.84-3.05 (7H, m), 3.26 (1H, dd, J=5.3Hz, J=11.9Hz)

Example 1

Preparation of 9-amino-8-fluoro-2,4-methano-1,2,3,4-tetrahydroacridin-1-ol:

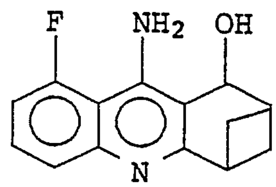

A solution (1M solution, 16 ml) of lithium aluminum hydride in tetrahydrofuran was added dropwise to a solution (100 ml) of ketone compound (3.57 g) obtained in Reference Example 4 in tetrahydrofuran, under ice-cooling. After stirring for 2 hours under ice-cooling, a saturated aqueous ammonium chloride solution was added dropwise to the reaction mixture. The mixture was extracted with ethyl acetate by salting-out techniques. The extract was washed with a saturated aqueous sodium chloride solution, and dried over anhydrous magnesium sulfate. Evaporation of the solvent gave a residue, which was subjected to column chromatography on silica gel. Elution with n-hexane-ethyl acetate (1:1) gave a crystalline product. The product recrystallized from ethyl acetate gave 9-amino-8-fluoro-2,4-methano-1,2,3,4-tetrahydroacridin-1-ol.
NMR ($CD_3OD$, TMS): 1.61-1.67 (1H, m), 1.91-1.98 (1H, m), 2.43-2.51 (1H, m), 2.61-2.69 (1H, m), 2.79-2.86 (1H, m), 3.21-3.27 (1H, m), 4.88 (3H, brs), 5.06 (1H, d, J=3.6Hz), 7.01-7.09 (1H, m), 7.46-7.58 (2H, m)

Example 2

Preparation of 9-amino-8-fluoro-2,4-methano-1,2,3,4-tetrahydroacridin-1-ol hydrochloride:

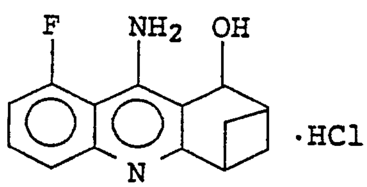

A solution of hydrogen chloride in ether was added dropwise to a solution of 9-amino-8-fluoro-2,4-methano-1,2,3,4-tetrahydroacridin-1-ol obtained in Example 1 in ethanol, until the solution was acidified. Evaporation of the resulting solvent gave a crystalline product, which was filtered with suction and then washed with ether, followed by drying to give 9-amino-8-fluoro-2,4-methano -1,2,3,4-tetrahydroacridin-1-ol hydrochloride, the melting point of which is 120-128 °C (decomposition).

Example 3

Preparation of 9-amino-2,3,5,6,7,8-hexahydro-1H-5,7-methanocyclopenta (b) quinoline:

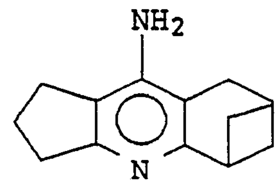

Under a nitrogen atmosphere, anhydrous aluminum chloride (1.2 g) was added to nitrobenzene (10 ml) at room temperature with stirring. 1-Amino-2-cyanocyclopenten-1 (0.8 g) and bicyclo [3,3,1] heptan-2-one (0.82 g) were added to the reaction mixture, followed by stirring for three hours at 110-120° C. A mixture of tetrahydrofuran (10 ml) and water (30 ml) was added dropwise to the reaction mixture under ice-cooling. An aqueous 10% sodium hydroxide solution was added dropwise to the mixture under ice-cooling, until the aqueous solution was alkalified. The mixture was extracted with ethyl acetate and washed with an aqueous 10% sodium chloride solution. After evaporation of ethyl acetate, 10% hydrochloric acid was added to the residue. The mixture was extracted with toluene. An aqueous 10% sodium hydroxide solution was added to the aqueous layer. The resulting mixture was alkalified and extracted with ethyl acetate, washed with an aqueous 10% sodium chloride, dried over anhydrous magnesium sulfate and evaporated. The residue was purified by column chromatography on silica gel to give 9-amino-2,3,5,6,7,8-hexahydro-1H-5,7-methanocyclopenta (b) quinoline.

NMR ($CDCl_3$, TMS): 1.48-1.56 (2H, m), 2.06-2.17 (2H, m), 2.42-2.49 (2H, m), 2.68-2.75 (4H, m), 2.86-2.96 (3H, m), 3.15-3.21 (1H, m), 3.89 (2H, brs)

Example 4

Preparation of 9-benzylamino-2,3,5,6,7,8-hexahydro-1H-5,7-methanocyclopenta (b) quinoline:

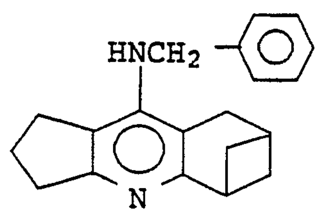

A mixture of 9-chloro-2,3,5,6,7,8-hexahydro-1H-5,7-methanocyclopenta (b) quinoline (1 g) obtained in Reference Example 5 and phenol (5 g) was heated at 80-90° C with stirring. Benzylamine (2.2 g) was added to the reaction mixture, followed by stirring for 6 hours at 130-140° C. An aqueous 20% sodium hydroxide solution was added to the reaction mixture under ice-cooling. The resulting mixture was alkalified and extracted three times with ethyl acetate. The extracts were combined and then washed twice with an aqueous 10% sodium hydroxide solution and a saturated aqueous sodium chloride solution, and dried over anhydrous magnesium sulfate. Evaporation of the solvent gave a residue, which was subjected to column chromatography on silica gel. Elution with ethyl acetate gave 9-benzylamino- 2,3,5,6,7,8-hexahydro-1H-5,7-methanocyclopenta (b) quinoline.

NMR ($CDCl_3$, TMS): 1.48-1.55 (2H, m), 1.96-2.07 (2H, m), 2.37-2.49 (2H, m), 2.65 (2H, d, J=2.6Hz), 2.82-2.90 (3H, m), 3.02-3.07 (2H, m), 3.15-3.21 (1H, m), 4.05 (1H, s), 4.65 (2H, d, J=5.9Hz), 7.27-7.40 (5H, m)

Example 5

Preparation of 9-phenethylamino-2,3,5,6,7,8-hexahydro-1H-5,7-methanocyclopenta (b) quinoline:

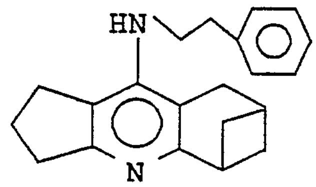

In the same method as described in Example 4, a mixture of 9-chloro-2,3,5,6,7,8-hexahydro-1H-5,7-methanocyclopenta (b) quinoline (1 g) obtained in Reference Example 5, phenethylamine (2.5 g) and phenol (5 g) was stirred for 6 hours at 130-140 °C and gave 9-phenethylamino-2,3,5,6,7,8-hexahydro-1H-5,7-methanocyclopenta (b) quinoline.

NMR ($CDCl_3$, TMS): 1.43-1.50 (2H, m), 2.01-2.12 (2H, m), 2.36-2.44 (4H, m), 2.78-2.92 (5H, m) 3.05 (2H, t, J=7.3Hz), 3.12-3.19 (1H, m), 3.66-3.76 (3H, m), 7.15-7.35 (5H, m)

## Claims

1. A pyridine derivative of the formula (I):

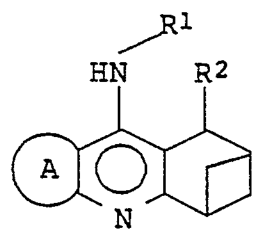

(I)

wherein $R^1$ is hydrogen or aralkyl, and $R^2$ and

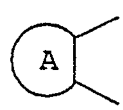

are defined as in the following (1) or (2):

(1) 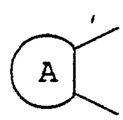 is 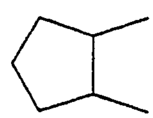 and $R^2$ is hydrogen; or

(2) 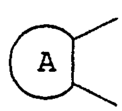 is 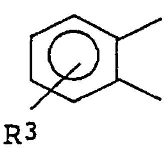

wherein $R^3$ is hydrogen, halogen or lower alkoxy, and $R^2$ is hydroxy; and pharmaceutically acceptable acid addition salts thereof.

2. A pyridine derivative according to Claim 1, wherein

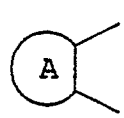 is 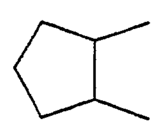

and $R^1$ is aralkyl.

3. A pyridine derivative according to Claim 1, wherein

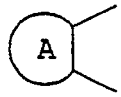 is 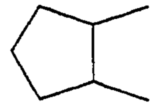

and $R^1$ is phenethyl or benzyl.

4. A pyridine derivative according to Claim 1, wherein

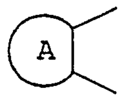 is 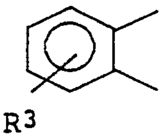 ,

$R^1$ is hydrogen, and $R^3$ is halogen.

5. A pyridine derivative according to Claim 1, wherein

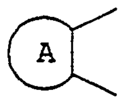 is 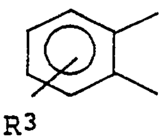 ,

$R^1$ is hydrogen, and $R^3$ is fluorine.

6. 9-amino-8-fluoro-1-hydroxy-2,4-methano-1,2,3,4-tetrahydroacridine and a pharmaceutically acceptable acid addition salt thereof.

7. A pyridine derivative according to any of Claims 1 to 6 for use as a pharmaceutically active substance.

8. Use of a pyridine derivative according to any of Claims 1 to 6 for the preparation of a pharmaceutical composition for the treatment of senile dementia of Alzheimer's type.

9. A pharmaceutical composition, which comprises at least one of the pyridine derivatives of formula (I) and a pharmaceutically acceptable acid addition salt thereof according to Claim 1 as an active ingredient together with a pharmaceutically acceptable carrier or diluent.

10. A pharmaceutical composition according to Claim 9, which is for the treatment of senile dementia of Alzheimer's type.

Claims for the following Contracting State : ES

1. A process for preparing a pyridine derivative of the formula (I):

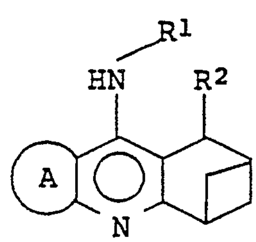

(I)

wherein $R^1$ is hydrogen or aralkyl, and $R^2$ and

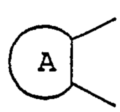

are defined as in the following (1) or (2);

(1) 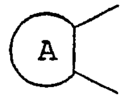 is 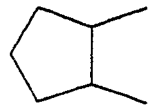 and $R^2$ is hydrogen; or

(2) 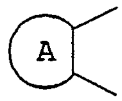 is 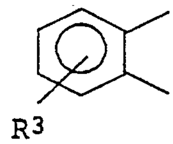

wherein $R^3$ is hydrogen, halogen or lower alkoxy, and $R^2$ is hydroxy; and pharmaceutically acceptable acid addition salts thereof,
which comprises
A) when

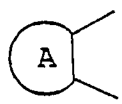 is 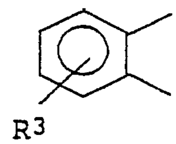 ,

wherein $R^3$ is hydrogen, halogen or lower alkoxy, and $R^2$ is hydroxy:

reducing compound (VII):

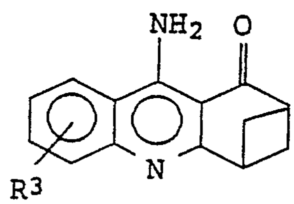

(VII)

either directly (when $R^1$ of formula (I) is hydrogen) in a manner known per se to obtain the pyridine derivative of the formula (I), wherein $R^1$ is hydrogen and $R^2$ is hydroxy,
or (when $R^1$ of formula (I) is aralkyl) first aralkylating compound (VII) in a manner known per se to obtain a compound of the formula (VIII):

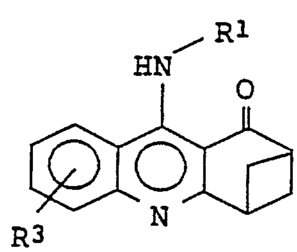

(VIII)

and then reducing compound (VIII) in a manner known per se to obtain the pyridine derivative of the formula (I), wherein $R^1$ is aralkyl and $R^2$ is hydroxy, or
B) when

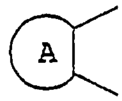 is 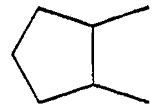

and $R^2$ is hydrogen:
aralkylating a compound of the formula (I′):

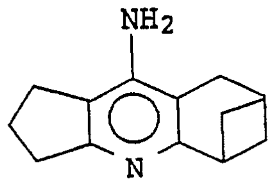
(I′)

(which compound (I′) corresponds to the pyridine derivative of the formula (I), wherein $R^1$ and $R^2$ are hydrogen),
directly in a manner known per se to obtain the pyridine derivative of the formula (I),
wherein $R^1$ is aralkyl and $R^2$ is hydrogen,
or first chlorinating compound (I′) in a manner known per se to obtain a compound of the formula (X):

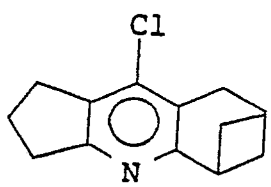
(X)

and then reacting compound (X) in a manner known per se with an amine of the formula (XI):
$R^4NH_2$ (XI)
wherein $R^4$ is aralkyl, to obtain the pyridine derivative of the formula (I), wherein $R^1$ is aralkyl and $R^2$ is hydrogen,
and optionally contacting the obtained pyridine derivative of the formula (I) in a manner known per se with an acid to obtain the pharmaceutically acceptable acid addition salt thereof.

2. A process according to Claim 1 for producing compounds of the formula (I),
wherein

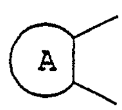 is 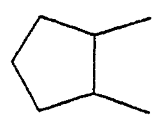

and $R^1$ is aralkyl.

3. A process according to Claim 1 for producing compounds of the formula (I),
wherein

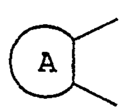 is 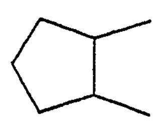

and $R^1$ is phenethyl or benzyl.

4. A process according to Claim 1 for producing compounds of the formula (I),
wherein

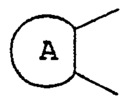 is 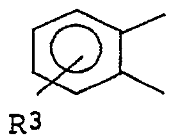 ,

$R^1$ is hydrogen, and $R^3$ is halogen.

5. A process according to Claim 1 for producing compounds of the formula (I), wherein

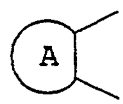 is 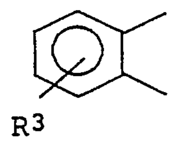 ,

$R^1$ is hydrogen, and $R^3$ is fluorine.

6. A process according to Claim 1 for producing the compound 9-amino-8-fluoro-1-hydroxy-2,4-methano-1,2,3,4-tetrahydroacridine or a pharmaceutically acceptable acid addition salt thereof.

Claims for the following Contracting State : GR

1. A pyridine derivative of the formula (I):

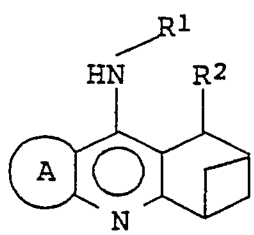

(I)

wherein $R^1$ is hydrogen or aralkyl, and $R^2$ and

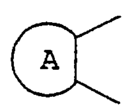

are defined as in the following (1) or (2):

(1) 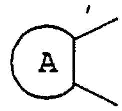 is 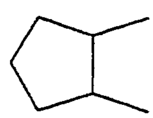 and $R^2$ is hydrogen; or

(2) 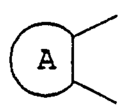 is 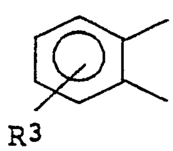

wherein $R^3$ is hydrogen, halogen or lower alkoxy, and $R^2$ is hydroxy; and pharmaceutically acceptable acid addition salts thereof.

2. A pyridine derivative according to Claim 1, wherein

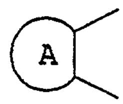 is 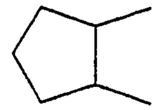

and $R^1$ is aralkyl.

3. A pyridine derivative according to Claim 1, wherein

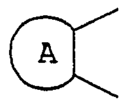 is 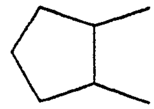

and $R^1$ is phenethyl or benzyl.

4. A pyridine derivative according to Claim 1, wherein

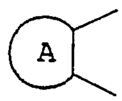 is 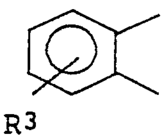 ,

$R^1$ is hydrogen, and $R^3$ is halogen.

5. A pyridine derivative according to Claim 1, wherein

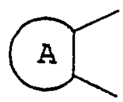 is 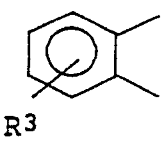 ,

$R^1$ is hydrogen, and $R^3$ is fluorine.

6. 9-amino-8-fluoro-1-hydroxy-2,4-methano-1,2,3,4-tetrahydroacridine and a pharmaceutically acceptable acid addition salt thereof.

7. A pyridine derivative according to any of Claims 1 to 6 for use as a pharmaceutically active substance.

8. Use of a pyridine derivative according to any of Claims 1 to 6 for the preparation of a pharmaceutical composition for the treatment of senile dementia of Alzheimer's type.

European Patent Office

# EUROPEAN SEARCH REPORT

Application Number

EP 90 10 7721

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 268 871 (SUMITOMO PHARMACEUTICALS CO. LTD.) * claims 1,18-25; table 6, compounds on page 23; last four compounds on page 24 * --- | 1-5,7-10 | C 07 D 219/10<br>C 07 D 221/16<br>A 61 K 31/44<br>A 61 K 31/47 |
| D,Y | US-A-4 735 953 (E.F. LAVRETSKAYA et al.) * column 2, lines 1-45 * --- | 1-3,7-10 | |
| D,Y | US-A-4 695 573 (G.M. SHUTSKE et al.) * claims 1,51,52,55-57,84; column 8, lines 52-68 *; & US-A-4631286 (cat. D); & US-A-4839364 (cat. D,P); ----- | 1,4,5,7-10 | |
| | | | TECHNICAL FIELDS SEARCHED (Int. Cl.5) |
| | | | C 07 D 219/00<br>C 07 D 221/00 |

The present search report has been drawn up for all claims

| Place of search | Date of completion of the search | Examiner |
|---|---|---|
| BERLIN | 25-07-1990 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)